Europäisches Patentamt

European Patent Office

Office européen des brevets

(11) Publication number: 0 262 651
A2

## (12) EUROPEAN PATENT APPLICATION

(21) Application number: 87114230.3

(22) Date of filing: 29.09.87

(51) Int. Cl.⁴: C12N 9/00 , C12N 9/28

(30) Priority: 30.09.86 US 913329

(43) Date of publication of application:
06.04.88 Bulletin 88/14

(84) Designated Contracting States:
AT BE CH DE FR GB IT LI LU NL SE

(71) Applicant: UNION CARBIDE CORPORATION
39 Old Ridgebury Road
Danbury Connecticut 06817(US)

(72) Inventor: Ananthapadmanabhan, Kavssery
Parameswaren
Apt. D, 150 Liberty Parkway
Spring Valley, New York 10977(US)
Inventor: Goddard, Errol Desmond
349 Pleasant Lane
Haworth New Jersey 07641(US)

(74) Representative: Weinhold, Peter, Dr. et al
Patentanwälte Dr. V. Schmied-Kowarzik
Dipl.-Ing. G. Dannenberg Dr. P. Weinhold Dr.
D. Gudel Dipl.-Ing. S. Schubert Dr. P. Barz
Siegfriedstrasse 8
D-8000 München 40(DE)

(54) Isolation of enzyme values from solution.

(57) This invention relates to a process for the recovery of enzymatic values from aqueous multi-phase systems containing polymeric materials which exhibit inverse solubility characteristics. Recovery is accomplished through elevating the temperature of said system above the point at which precipitation of the polymeric material occurs but below the temperature where undue degradation of the enzyme values occurs. The polymeric precipitate may then be removed through conventional techniques.

EP 0 262 651 A2

## ISOLATION OF ENZYME VALUES FROM SOLUTION

This invention relates to a process for the recovery of enzyme values from aqueous multiphase systems. More particularly, it relates to a process for the recovery of enzyme values from aqueous solutions containing polymeric materials, such as polyalkylene glycols, which exhibit inverse solubility characteristics. Precipitation of the polymeric materials may be accomplished through a temperature induced decrease in its solubility, thereby allowing its removal from the system and the isolation of the enzyme-containing aqueous solution.

The potential applications for biologically active proteins, such as enzymes, have greatly increased. Commercial implementation of this technology now frequently depends on the ability to isolate these substances at reasonable cost. Until recently, separation technology which could support industrial applications was limited to filtration and centrifugation. However, these techniques are extremely dependant upon particle size and therefore approach their limit of usefulness during the harvest of even small intact microorganisms. The problems encountered are therefore greatly increased during the attempted isolation of intracellular components from ruptured cells where component size is, of course, greatly reduced.

The process of affinity partitioning using two phase aqueous systems has previously been employed in enzyme isolations. For instance, U.S. Patent No. 4,144,130 discloses its use for the isolation of enzymes from other intracellular matter. U.S. Patent No. 4,343,735 discloses the use of affinity partitioning in the recovery of interferon. U.S. Patent No. 4,207,200 discloses the isolation of deoxyribonucleic acid, while U.S. Patent No. 4,470,969 discloses the use of this technique in the recovery of human coagulation factors VII and VII-VII. Affinity partitioning basically involves the formation of multiple, distinct phases in a common solvent following the addition of materials, such as polymers, which produce immiscible phases when in solution and the selective affinity of a molecule for one phase over the other. Affinity partitioning systems having an aqueous solvent has been known since the late nineteenth century from the work of Beijerinck who published his findings regarding aqueous phase formation with agar and gelatin. Unlike conventional techniques such as filtration and centrifugation, affinity partitioning offers the potential of improved recovery of cellular components in industrial scale recoveries since it is not dependant upon particle size.

Once biological material such as enzymes has been concentrated in one phase through the use of affinity partitioning, its isolation from that phase must still be accomplished. In the recovery of enzymes where aqueous solutions of polyalkylene glycols, such as polyethylene glycol, are typically employed in conjunction with either aqueous dextran or inorganic salt solutions, this usually entails physical removal of the enzyme from the polyalkylene glycol phase in which the enzyme is usually concentrated. One method of accomplishing this is through use of ion-exchange columns. However, this method is capital intensive and is often time consuming. Co-pending applications, U. S. Serial Nos. 728,241 and 728,242 disclose alternative procedures for the isolation of enzymes from phase-forming materials, namely the formation of enzyme containing precipitates.

However, utility of enzymes often requires their presence in solution. Therefore, the above described process wherein ion exchange columns are utilized for enzyme separation would then require removal of the enzymes from the ion exchange column and their resuspension. The precipitates produced through the above-mentioned processes would also require disassociation of the enzyme-containing precipitates and the resuspension of enzyme.

Applicants have, through the instant invention, sought to provide a process for the concentration and isolation of enzyme values of an aqueous multi-phase system without the need to remove the enzymes from solution thereby avoiding the additional processing associated with resuspension of the enzymes. This goal is accomplished through the use of aqueous affinity partitioning systems and removal of the polymeric materials present in at least one phase of these systems, thereby leaving the enzyme suspended in the aqueous solution.

This invention provides the isolation and recovery of enzymes from at least one phase of an aqueous multi-phase system containing a polymeric component which exhibits inverse solubility characteristics.

More particularly, the present invention relates to a process for the recovery of enzyme values from aqueous solutions containing polymeric materials which exhibit inverse solubility characteristics through the temperature induced precipitation of polymeric materials, thereby allowing easy isolation of the enzyme-containing aqueous solution.

For the purposes of the invention, the phrase "inverse solubility" means the solubility of the polymeric materials varies inversely to solution temperature, i.e. their solubility decreases with increased solution temperature. The solubility of most solutes varies directly with solution temperature. Inverse solubility is therefore directly opposed to the effect exhibited by most solutes.

The instant invention may generally be applied to the isolation of any enzymes from an aqueous enzyme-containing solution containing polymeric material which exhibits inverse solubility characteristics. Thus, the identity of the enzymes which are recoverable through use of the instant processes is not critical. Any enzyme may be employed so long as the enzyme does not suffer an unacceptable loss in activity due to the elevated temperatures and, optionally, the presence of inorganic salts which characterize this invention. Preferred enzymes are proteolytic enzymes and amylases.

Proteolytic enzymes are those which selectively hydrolyze -CO-NH-linkages. These include exopeptidases, which selectively act upon terminal amino groups of a peptide chain, and endopeptidases, which are indiscriminant in the location of their action, and mixtures thereof. They may originate from plants, animals or procaryotic or eucaryotic microbes. Representative of individual species of proteolytic enzymes include pepsin, resin, trypsin, chymotrypsin, pankrin, enterokinase, papain, chymopapain, ficin, bromelin, B. subtilis proteinase, insulinase, Aspergillus proteinase, carboxypeptidase, protaminase, asparaginase, fungal penicillin amidase, bacterial penicillinase and mixtures thereof. Most preferred of the proteases are alkaline proteases, which exhibit maximum activity under neutral and basic pH conditions. Commercially available alkaline proteases include Alkalase™ manufactured by Novo and the Milezyme™ ApL series, manufactured by Miles Laboratories.

Amylases are enzymes which act through endohydrolysis of alpha-1,4-D-glucoside bonds of polysaccharides having more than three adjacent alpha-1,4-D-glucose units. Amylases may be derived from plant, animal, fungal or bacterial sources. Examples are Cereal beta amylase (wheat and barley malt), pancreatic amylase (bovine/porcine), fungal alpha-amylase (Aspergillus spp.) and thermostable alpha-amylase (Bacillus licheniformis), respectively. Commercially available amylases include Aquazym™ manufactured by Novo.

The solubility of compounds in an aqueous medium is affected by the temperature of the medium. As discussed above, the solubility in aqueous media of most compounds varies directly with the temperature of the medium. The solubility of certain polymeric materials however, such as polyalkylene glycols, poly(oxyalkylene) polymers, poly(oxyalkylene) copolymers and polyvinyl pyrrolidone, has surprisingly been found to vary inversely with the temperature of the aqueous medium in which they are contained. Therefore, by increasing the temperature of enzyme-containing aqueous solutions containing these polymeric materials, the polymeric materials be can effectively precipitated out of solution. The precipitate can then be readily removed from the solution, leaving an aqueous enzyme-containing solution substantially free of the polymeric material, thereby avoiding the necessity of resuspending the enzyme.

Examples of polymeric materials which possess inverse solubility include polyalkylene glycols, poly(oxyalkylene) polymers, poly(oxyalkylene) copolymers, ethoxylated surfactants, silicone modified polyethers and polyvinyl pyrrolidone.

Through use of the term polyalkylene glycol, what is meant is both simple and substituted polyalkylene glycols. The following compounds are therefore included within the meaning of this term and the scope of the present invention: polyethylene glycol, polypropylene glycol, acrylic acid substituted polyethylene glycol, methacrylic acid substituted polyethylene glycol, methoxy-capped and ethoxy-capped polyethylene gylcols. These glycols typically have average molecular weights ranging from about 200 to about 20000. Preferred are polyethylene glycols having average molecular weights ranging from about 1000 to about 10000, most preferably about 1400 to about 8000.

Through the terms poly(oxyalkylene) polymers and poly(oxyalkylene) copolymers, what is meant are polymers and copolymers of polyoxyalkylenes having average molecular weights ranging from about 200 to about 30,000. The following compounds are therefore included within the meaning of this term and the scope of the present invention: ethylene oxide-propylene oxide copolymers, grafted copolymers, end capped copolymers. Preferred are ethylene oxide-propylene oxide copolymers having average molecular weights ranging from about 200 to about 20,000, most preferably about 1,000 to about 10,000.

Through use of the term "ethoxylated surfactants" what is meant is molecules containing both ethylene oxide moieties and methylene groups. Examples of these materials include ethoxylated nonylphenols and ethoxylated alcohols having hydrocarbon chains containing from about 8 to 24 carbon atoms.

Through use of the term "silicone-modified polyethers" what is meant is the reaction product of polyoxyalkylenes and silanes or siloxanes. Examples of these materials include poly(oxyalkylene) modified dimethyl siloxanes.

Also within the scope of this invention is the isolation of enzyme values from aqueous polyvinyl pyrrolidone-containing aqueous solutions.

It has been further found that while polymeric materials which exhibit inverse solubility characteristics may indeed be precipitated out of an aqueous solution through simple elevation in temperature, the rise in temperature needed to accomplish this may well exceed the temperature at which the activity of the enzyme contained therein in unduly effected, such through its denaturation . In order to minimize enzyme deactivation, small amounts of inorganic salts should be present in the solution when necessary, thereby decreasing the temperature to which the solution must be raised in order to bring about precipitation of the polymeric material. Suitable salts include sodium sulfate, sodium phosphate, magnesium sulfate, magnesium phosphate, potassium sulfate and potassium phosphate. While these salts need only be present in amounts which will lower the precipitation temperature, the addition of increased amounts will increasingly lower the temperature at which precipitation of the polymeric material occurs.It is therefore possible to realize significant energy savings through addition of inorganic salts. The upper limit of inorganic salt concentration is however limited as the addition of excess inorganic salt may cause the undesirable formation of an additional aqueous two-phase system. Salt concentration must therefore be maintained below this level. Salt concentration should be maintained in the range that will cause the formation of a two-phase system. Generally, this is in the range of from about 0.1 to about 10.0 weight percent of the solution, preferably from about 2 to about 8 weight percent of the solution.

It should be here noted that the presence of inorganic salt often does not require its addition, as polymer-inorganic salt multi-phase aqueous systems are known and are often employed in the isolation of enzyme values, as discussed more fully below. Adequate concentrations of inorganic salts may therefore already be present in the enzyme-containing solution to effect significant lowering of the precipitation temperature of the polymeric material.

As noted above, affinity partitioning employs a system having at least two immiscible phases and a common solvent. A number of systems wherein water is the common solvent and therefore suitable for the separation of biopolymer are known, for instance those described in Albertsson, P. A., Partition of Cell Particles and Macromolecules, Uppsala, lst edition (1960), 2nd edition (1971) and U.S. Patent No. 3,897,414.

Those aqueous multi-phase systems which are suitable for use in conjunction with the instant invention are those in which the enzymes will be concentrated in an aqueous phase containing those materials which exhibit inverse solubility characteristics, such as polyalkylene glycols, poly-(oxyalkylene) polymers, poly(oxyalkylene) copolymers, polyvinyl pyrrolidone or mixtures thereof.

Illustrative examples of two phase aqueous systems for use in practicing the present invention may be used include: polyethylene glycol/dextran sulphate, charged polyethylene glycol/dextran, dextran/polyethylene glycol, polypropylene glycol/methoxypolyethylene glycol, polypropylene glycol/polyethylene glycol, polypropylene glycol/polyvinyl alcohol, polypropylene glycol/polyvinyl pyrrolidone, polypropylene glycol/hydroxypropyldextran, polypropylene glycol/dextran, polyethylene glycol/polyvinyl alcohol, polyethylene glycol/polyvinyl pyrrolidone, polyethylene glycol/water-soluble copolymer of sucrose and epichlorohydrin polyethylene glycol/water-soluble starch and polyethylene glycol/glycogen.

These aqueous systems may also contain additional materials including pH modifiers, such as sodium hydroxide and hydrochloric acid, and organic solvents such as propylalcohol, glycerol or 2-butoxyethanol.

The preferred aqueous systems are 1) systems comprising from about 1 to about 30 wt. % polyethylene glycol having an average molecular weight of about 1000 to about 10000 and from about 3 to about 30 wt. % dextran, 2) systems comprising from about 3 to about 50 wt. % polyethylene glycol having an average molecular weight of about 1000 to about 10000 and from about 3 to about 25 wt. % inorganic salts, and 3) UCON FLUIDS (ethylene oxide-propylene oxide) copolymers with an average molecular weight of 200-20000 and 3 to 30% inorganic salts. Inorganic salts preferably used in these preferred systems are magnesium sulfate, potassium sulfate, sodium phosphate, sodium sulfate and sodium chloride in amounts of from 0.1 to 10 wt. %, most preferably 0.5 to 5 wt. %.

The enzyme source, whether an aqueous enzyme-containing solution or a broth containing fragmented cells, is simply contacted with the aqueous partitioning system accompanied with sufficient agitation to disrupt the phases of the system. The system is then left undisturbed to allow for reestablishment of distinct phases. The pH of the system should be maintained at a level at which undue denaturation of the enzyme does not occur and concentration of the enzyme in the polymer material-containing phase is enhanced. This will of course vary with the enzyme employed. In general, the pH of the system should be maintained in the range of about 4 to about 10. In the case of the preferred enzymes, alkaline protease and alpha-amylase, the pH of the system is preferably maintained in the range of about 5 to about 8.

Temperatures are also not critical at this point but should be maintained at a point below which both denaturation of the enzyme and precipitation of the polymeric materials do not occur. The temperature of the system should also be maintained at a point above which fluidity of the system is affected. After reestablishment of the phase structure of the system, the enzyme-containing polyalkylene glycol phase is then isolated.

The temperature of the isolated polyalkylene glycol-containing phase is then elevated through the application of an external heat source. The temperature may be raised to any level at which precipitation of the polymeric materials occurs while deterioration of the components does not occur. Temperatures at which undue denaturation of the enzyme occurs should, of course, be avoided. Temperatures should generally be maintained below about 90° C. This will, of course, vary with the particular enzyme and polymeric materials employed. In the case of the preferred enzymes, alkaline protease and alpha-amylase, the temperature of the system is preferably maintained in the range of about 50 to about 75° C.

While the temperature of the isolated phase is still elevated, the precipitate of polymeric materials should be separated from the aqueous enzyme-containing phase. This may be accomplished through simple decanting of the aqueous supernatant, filtration or other well known physical means.

The following examples illustrate embodiments of the present invention and are not to be construed as a limitation of its scope.

These examples are offered to demonstrate isolation of an enzyme through use of the present invention and retention of activity of the enzyme so isolated.

All parts and percentages are by weight unless otherwise indicated. All temperature values are given in degrees Celsius.

EXAMPLE 1

Into a 15 ml. Pyrex centrifuge tube was introduced

a) 2.5 grams of an aqueous 40 wt.% solution of a ethylene oxide-propylene oxide (1:1) copolymer having an average molecular weight of about 4300, manufactured by Union Carbide Corporation and marketed under the designation UCON™ fluid 50 HB 5100,

b) 1 gram of 0.5 wt. % solution of $^\lambda$-amylase marketed by Miles Laboratories under the designation, and

c) distilled water to bring the solution up to 10.0 grams total weight.

The contents of the centrifuge tube were then mixed for a period of ten minutes.

This solution represented an aqueous poly-(alkylene) oxide, enzyme-containing phase as would be obtained by isolating one phase of a typical affinity partitioning system.

The centrifuge tube was then placed for two hours in an oven which had been preheated to 80° C. At the end of this period, it was noted that the centrifuge tube contained a phase containing a cloudy precipitate and a clear aqueous phase. The aqueous phase was removed through the use of a syringe. The recovered filtrate was then set aside to cool to room temperature.

The concentration of alpha-amylase contained in the filtrate was then determined through the following method.

Standard solutions of $^\lambda$-amylase at concentrations varying from about 0.0 to about 0.2 were prepared. The ultra violet absorbance of these solutions at 280 nanometers was then calculated. From this data, an absorbance curve for $^\lambda$-amylase was composed.

It was found through comparison with the above described absorbance curve that the concentration of the $^\lambda$-amylase in the recovered filtrate was 0.031 wt. % which represents a recovery of 62 wt.%.

This procedure was repeated with a recovery of 0.023 wt. % of alpha-amylase or 46 wt.%.

EXAMPLE 2

The procedure set forth in Example 1 was repeated except that the initial concentration of enzyme was increased to 0.1% through the use of 2.0 grams of the 0.5% aqueous solution.

It was found that the concentration of the 2-amylase in the recovered filtrate was 0.065 wt. % which represents a recovery of 65 wt.%.

This procedure was repeated with a recovery of 0.061 wt. % of alpha-amylase or 61 wt. %.

EXAMPLE 3

The procedure set forth in Example 1 was repeated except that the initial concentration of enzyme was increased to 0.15% through the use of 3.0 grams of the 0.5% aqueous solution.

It was found that the concentration of the $^\lambda$-amylase in the recovered filtrate was 0.101 wt. % which represents a recovery of 67 wt. %.

This procedure was repeated with a recovery of 0.094 wt. % of $^\lambda$-amylase or 63 wt. %.

## Claims

1. A method for recovering enzymatic values from an enzyme containing aqueous solution which contains at least one polymeric material which exhibits inverse solubility characteristics comprising

(a) elevating the temperature of said solution above the temperature of precipitation of said polymeric material but below the temperature where degradation of any component of said solution occurs, and

(b) separating the polymeric precipitate from the enzyme-containing solution.

2. The method of Claim 1 wherein the enzyme is selected from proteolytic enzymes and amylases.

3. The method of Claim 2 wherein the proteolytic enzyme is alkaline protease.

4. The method of Claim 2 wherein the amylase is $\lambda$-amylase.

5. The method of any of Claims 1 to 4 wherein the polymeric material which exhibits inverse solubility characteristics is selected from polyalkylene glycols, poly(oxyalkylene) polymers, poly(oxyalkylene) copolymers, ethoxylated surfactants, silicone-modified polyethers and polyvinyl pyrrolidone.

6. The method of Claim 5 wherein the polyalkylene glycol is selected from polyethylene glycol, polypropylene glycol,acrylic acid-substituted polyethylene glycol, methacrylic acid-substituted polyethylene glycol, methoxy-capped polyethylene glycol, and ethoxy-capped polyethylene glycol.

7. The method of any of Claims 5 or 6 wherein the polyalkylene glycol has an average molecular weight of about 200 to about 20,000, particularly of about 1000 to about 10,000, and most preferably of about 1400 to about 8.000.

8. The method of any of Claims 5 to 7 wherein the polyalkylene glycol is polyethylene glycol having an average molecular weight of about 1000 to about 10,000.

9. The method of Claim 5 wherein poly(oxyalkylene) polymers comprise copolymers of ethylene oxide and propylene oxide having average molecular weights of about 200 to about 30,000, particularly of about 200 to about 20,000, and most preferably of about 1,000 to about 10,000.

10. The method of Claim 5 wherein the ethoxylated surfactants are selected from ethoxylated nonylphenols and ethoxylated alcohols containing hydrocarbon chains of from about 8 to 24 carbon atoms, and the silicone-modified polyethers comprise poly(oxyalkylene)-modified dimethyl siloxanes.

11. The method of Claim 5 wherein the polymeric material comprises polyvinyl pyrrolidone.

12. The method of any of Claims 1 to 11 wherein the temperature of the aqueous solution is elevated to less than about 90°C, e.g. to between about 50° and about 75°C.

13. The method of any of the Claims 1 to 12 wherein the enzyme-containing aqueous solution is provided with inorganic salts in a concentration of from about 0.1 to about 10.0 wt. %, particularly from about 2 to about 8 wt. %, prior to elevation of the temperature of said solution.

14. The method of Claim 13 wherein the inorganic salts are selected from sodium sulfate, sodium phosphate, magnesium sulfate, magnesium phosphate, potassium sulfate and potassium phosphate.

15. A method for recovering enzymatic values from an aqueous polyethylene glycol-containing solution containing $\lambda$-amylase or alkaline protease comprising

(a) providing said solution with inorganic salts in a concentration of from about 0.1 to about 10.0 wt. %,

(b) elevating the temperature of said solution to a temperature of about 50°C to about 90°C , and

(c) separating the enzyme-containing solution from the polyethylene glycol precipitate.